# EUROPEAN PATENT SPECIFICATION

(11) **EP 2 872 041 B1**
(45) Date of publication and mention of the grant of the patent: **03.07.2019**
(21) Application number: 13741818.2
(22) Date of filing: 15.07.2013
(51) Int. Cl.: A61B 5/11, A61B 5/00, G16H 50/20

(54) **DEVICE TO DETERMINE DYSKINESIA**
VORRICHTUNG ZUR BESTIMMUNG VON DYSKINESIE
DISPOSITIF DESTINÉ À DÉTERMINER LA DYSKINÉSIE

(30) Priority: 13.07.2012 GB 201212544
(43) Date of publication of application: 20.05.2015
(73) Proprietor: Clearsky Medical Diagnostics Limited, Heslington York North Yorkshire YO10 5GA (GB)
(72) Inventor: SMITH, Stephen, York Yorkshire YO10 5DD (GB)
(74) Representative: ip21 Ltd
(86) International application number: PCT/GB2013/051888
(87) International publication number: WO 2014/009757

(56) References cited:
- WO-A1-2012/098388
- US-A1- 2005 234 309
- US-B1- 8 187 209
- STEPHEN L SMITH ET AL: "Diagnosis of Parkinson's disease using evolutionary algorithms", GENETIC PROGRAMMING AND EVOLVABLE MACHINES, KLUWER ACADEMIC PUBLISHERS, BO, vol. 8, no. 4, 4 October 2007 (2007-10-04) , pages 433-447, XP019551470, ISSN: 1573-7632, DOI: 10.1007/S10710-007-9043-9
- JI-WON KIM ET AL: "Quantification of bradykinesia during clinical finger taps using a gyrosensor in patients with Parkinsonâ s disease", MEDICAL & BIOLOGICAL ENGINEERING & COMPUTING, SPRINGER, BERLIN, DE, vol. 49, no. 3, 30 October 2010 (2010-10-30), pages 365-371, XP019886771, ISSN: 1741-0444, DOI: 10.1007/S11517-010-0697-8

## Description

### Field of the Invention

The present invention relates to a device to determine the presence and type of dyskinesia exhibited by a patient The device can be worn by the patient without interrupting their daily routine.

### Background to the Invention

Clinical Management of Levodopa-induced Dyskinesia (LID) is one of the most challenging problems in the treatment of Parkinson's disease (PD). One of the main issues is that patients and clinicians frequently fail to document in detail when dyskinesias are occurring and hence attempts to alter medications may not be well-informed and lead to little improvement, or even worsening of the problem.

Inaccurate recording of dyskinesias may occur for a number of reasons. Firstly, there are several sub-types of dyskinesia e.g. choreiform movements occurring at peak dose, dystonia occurring with trough doses and diphasic dyskinesia that occurs both when plasma drug doses rise and also as they fall. This makes it very difficult for physicians, and certainly for patients, to document the character and timing of each symptom in detail. For example, peak dose dyskinesia may look similar to PD tremor - both are involuntary movements - but the former is due to high drug levels (*on*) and the latter to sub-therapeutic levels (*off*). Incorrect labelling of an involuntary movement may lead therefore to inadvertent worsening of the problem if the medication doses are adjusted in the wrong direction. Furthermore patients with advanced PD may move quickly from an off tremor state to an *on* dyskinetic state, further complicating this issue.

Second, even if the patient and/or clinician correctly recognise different types of dyskinesia they rarely are able to document the timing of these periods accurately for prolonged periods without considerable interruption to the patient's daily activities. If the timing of symptoms is not known, the treating physician will have difficulty deciding which particular doses need to be altered. Some patients are able to meticulously document their symptoms themselves for several days to help the physician but this is clearly rather onerous and an electronic device that is able to record this information would be much easier for the patient, and more accurate for the physician. If the dyskinesias are particularly troublesome and attempts to manage them as an outpatient are not succeeding, patients may need to be admitted to the ward for detailed monitoring. This involves a specialist nurse, or a junior doctor, assessing the patient every hour or so and recording this information on a chart for the consultant to then use to guide treatment decisions. This is clearly an expensive and time consuming method but may be the only way to manage patients with complicated symptoms and drug regimes.

Third, it is important to highlight that drug regimes in PD are rarely straightforward by the time dyskinesias have become problematic - i.e. typically patients will be on 2 or more types of drugs - usually a levodopa based drug (such as Sinemet or Stalveo) which may be taken 3 to 6 (or even 10 in advanced disease) times a day and a dopamine agonist drug (such as Pramipexole or Ropinerole) typically taken 1-4 times a day so the statement that physicians may simply alter the drug depending on the dyskinesia is an over simplification. Typically patients will have periods of dyskinesia when *on,* alternating with periods of being *off.* The medications given prior to the periods of peak dose dyskinesia will need to be lowered or the frequency of doses reduced, and those given prior to the period of being *off* will need to be raised. This is clearly quite a delicate task when up to 10 doses of medications are being used over a 24 hours period, and accurate recording of exactly when dyskinesia is occurring is of paramount importance.

Fourth, technological methods for recording paroxysmal symptoms are used in other areas of neurology (e.g. video telemetry for patients with epilepsy) and medicine (Holter monitor recording for paroxysmal arrhythmias in cardiology) but currently there is no equivalent method for dyskinesia in Parkinson's disease. This is surprising since PD is a fairly common condition, affecting approximately 1% of those aged over 60 years old: and dyskinesias develop in approximately 40% of PD patients taking levodopa for 4-6 years and in almost 90% of those treated for 10 years.

### Summary of the Invention

According to the invention there is provided a device to determine the extent of dyskinesia in a subject, the device comprising a sensor, removably attachable to the subject's limb, torso or head, said sensor including a first detector to detect 3-D motion, data retention means to retain data generated by the detector;
transfer means to transfer said data to the data retention means;
processing means, to process the data in the data retention means and classify said processed data;
output means to display the diagnosed condition to a user;
wherein the processing means employs an evolutionary algorithm in the classification of said data and comparison with a known dyskinesia condition,
the device further including a second motion detector to detect pitch, roll and yaw of the device.

The device allows constant monitoring of a subject to take place, without the need for a clinician to be present, and for accurate analysis to be rapidly made of the subject's condition. This represents a time saving for the clinician, and can also, due to the non-invasive nature of a sensor causes less distress to the subject.

Conveniently, the first motion detector comprises one or more accelerometers to provide 3-D motion data.

Conveniently, the device includes a further motion detector to measure position data, further conveniently the position data is determined with reference to Cartesian co-ordinates, which position data can be processed by the processing means to yield velocity and/or acceleration data.

Preferably the transfer means is a wireless transmission means to a remote data retention means which allows for a smaller device to be worn by the user, said device not having to include processing elements and also allowing for a subject to be assessed whilst at home, with data being processed centrally at a hospital or clinic.

Advantageously, the device comprises a plurality of sensors.

Preferably, the or each sensor obtains data readings at a rate of from 10-200Hz, and especially preferably at around 100Hz.

### Brief Description of the Drawing

The invention is described with reference to the accompanying drawing which shows by way of example only, one embodiment of a device. In the drawing:

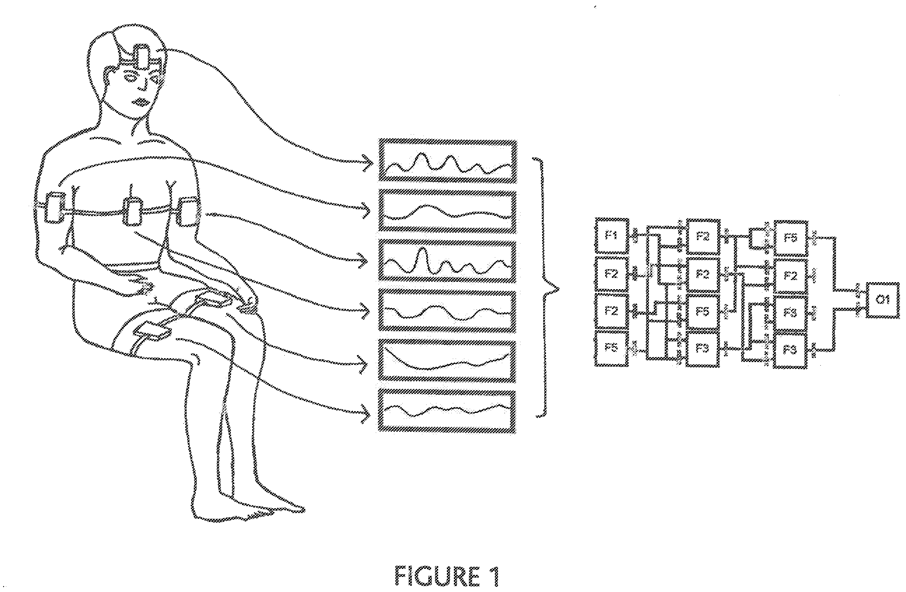

Figure 1 illustrates the sampling and processing of data from a subject.

### Detailed Description of the Invention

Although Parkinson's disease can be treated through the oral administration of the drug Levodopa, it is very important that the dose administered be the correct one. Both a deficiency and an excess of Levodopa can result in the patient suffering unwanted effects, The situation is complicated by a number of factors. Firstly, there is the general concern as to whether the who may well be in a confused state of mind, is taking the prescribed medication correctly. Second, the effects of a wrong dosage can very often, especially to the untrained eye be indistinguishable from one another, It is certainly not unknown, for example, for individuals whose Levodopa levels were too high, to be misdiagnosed with the opposite problem and given additional doses of the drug and for patients with too low a level to have the drug withheld.

Often the only way of assessing accurately the state of a patient is for dose monitoring in a hospital under the detailed supervision of a qualified physician.

The present invention provides a device for monitoring the movement of a patient which device includes a means for effectively determining the symptoms displayed by the patient and also of clarifying said symptoms to provide an assessment of the patient. This then enables the correct treatment to be carried out to address the second of the above problems and in many instances avoid the first problem.

The invention contemplates in its broadest aspect a device for determining dyskinesia, including the type of dyskinesia being exhibited by a The type of dyskinesia is related to levels of Levodopa within the patient's body, A device, or plurality of devices records the movements of the subject under investigation. The movements are then analysed for given markers which are indicative of the particular dyskinesia type.

In one embodiment of the invention, the subject patient wears a number, typically eight sensors, about their limbs, and also attached to their head and trunk. These record the direction, velocity, or acceleration of the limb. Data can thus be collected on a continuous and automatic basis without discomfort to the subject, who can be in their normal environment, and also without the need for a medical practitioner to be present to observe the subject.

As illustrated with respect to Figure 1, the sensors are wireless accelerometer gyroscopes which are small enough to be strapped to and worn by the subject. Attachment to the patient can be by conventional means, such as Velcro™. The sensors include six measuring means to measure movement in three mutually perpendicular directions x, y, z along with pitch, roll and yaw, (i.e. rotation about each of those three directions). Data is sampled in accordance with a timing mechanism within the sensor at a rate of about 100Hz. Other data collection rates can be contemplated such as 10, 20, 60Hz, along with 120, 150, 200Hz etc. The skilled person will make the decision as to the rate to use based on the desired accuracy and the memory capacity available for the data, increasing the amount of data seat will usually require a greater data storage capacity.

Any data which needs to be transmitted can be sent via, for example, Bluetooth to a Smartphone. Alternative means can of course be employed. If analysis means are not included with each sensor then processing can be carried out remotely.

An example of processed data is as follows. Firstly, speed data on the movement of an individual sensor can be obtained from accelerometer derived data or other position data, from the application of geometry. So for example, the distance d moved during two different data sampling times (t-1) and t, is d=sqr[(xₜ-xₜ₋₁)²+(yₜ-yₜ₋₁)²+(zₜ-zₜ₋₁)²], where x, y and z are expressed in Cartesian co-ordinates. This translates to a speed of d/ Δt where At is the time between t and t-1, For a sampling rate of 100Hz Δt will be 0.01s.

in addition angular rotation can be assigned with value : abs (rollₜ - rollₜ₋₁) + abs (pitchₜ - pitchₜ₋₁) + abs (yawₜ-yawₜ₋₁) where abs indicates the operator, 'take absolute value'.

Once calculated the speed data is presented to an analyser means to determine the type of dyskinesia being exhibited by the subject.

The analyser means employs an evolutionary analysis methodology as disclosed in GB 1100794.5 (published as WO 2012/098388 A1 on 26th July 2012, and therefore only of relevance under Article 54(3) EPC) in order to aid in the decision making process. As such an initial training stage for the decision making process is used in which data from each device is passed to and processed by the methodology to predict one of the five conditions previously set down in the methodology, and is detailed below. Again as further conditions are identified, these can be included in the methodology.

The algorithm underlying the evolutionary analysis is executed a number of times. Each execution produces one or more classifiers. An ensemble classifier is then created by selecting a subset of maximally-diverse classifiers from those found during all executions of the evolutionary algorithm. This selection of maximal-diversity can be achieved either by
(i) carrying out different runs of the evolutionary algorithm on different subsets of the data, or
(ii) by post-hoc analysis, where the behaviour of each classifier is explicitly measured and those with minimal behavioural overlap are chosen for the ensemble. Behaviour, in this sense, can either be the differential response of the classifier to different subsets of the data, or the classifier's ability to recognise particular patterns within the data.

In addition, angular rotation data is similarly processed through a second evolutionary analysis methodology and again the results compared with previously set down conditions to provide an assessment of the subject.

The data from the speed and the angular rotation classifier can be combined together in an ensemble classifier analysis methodology to yield higher accuracy in reaching a conclusion. In addition, an artificial biological network (ABN) can be employed in combination with the evolutionary algorithm and an ensemble classifier.

One means of producing classifications to which the results of analysed data can be fitted, and which in particular can be used in the training stage of the analysis methodology, involves using one or more trained clinicians to carry out the assessment. For example, the clinicians can assign a value of, for example, 1-4 against each of several types of different dyskinesia it will be recognised that a broader value range can be used, although the difficulty in assessing which of a then narrower value to assign would increase. For example, the four categories which could be used are 1) minimal 2) mild, 3) moderate, 4) severe / incapacitating. As examples of overt dyskinesia which can be classified in this manner are a) choreiform dyskinesia, b) dystonic dyskinesia, c) other dyskinesia, d) tremor, e) bradyskinesia. Again, it will be recognised that other dyskinesias can also be characterised as will be apparent to the skilled person, Moreover, the difference between dyskinesia (involving the side effect of the medication Levodopa) and the symptoms of Parkinson's Disease.

Once an evolutionary Algorithm has been tried to recognise dyskinesia, such as bradykinesia (the main symptom of Parkinson's Disease, a slowing of movement) and Parkinsonian rest tremor, the evolved expression is examined to identify those common aspects of all the subject patient's movement disorders that contributed most to the expression. A second evolutionary algorithm can be trained on those specific aspects to evolve a yet more discriminating expression.

It will of course be understood that the invention is not limited to the specific details described herein, which are given by way of example only, and that various modifications and alterations are possible within the scope of the invention.

## Claims

1. A device to determine the extent of dyskinesia in a subject, the device comprising a sensor removably attachable to subject's limb, torso, or head, said sensor including a first detector to detect a 3-D motion,
data retention means to retain data generated by the detector;
transfer means to transfer said data to the data retention means;
processing means, to process the data in the data retention means and classify said processed data;
output means to display the diagnosed condition to a user; **characterised in that** the processing means is adapted to employ an evolutionary algorithm in the classification of said data and comparison with a known dyskinesia condition, and **in that** the sensor further includes a second motion detector to detect pitch, roll and yaw of the sensor, said second motion detector providing further data to the data retention means, to also be used by the processing means to determine the extent of dyskinesia.

2. A device according to claim 1, wherein the first motion detector comprises one or more accelerometers to provide said 3-D motion data.

3. A device according to any preceding claim, wherein the device includes a further motion detector to measure position data.

4. A device according to 3 wherein the position data is determined with reference to Cartesian co-ordinates.

5. A device according claim 3 or 4 wherein said processing means converts position data to velocity and/or acceleration data.

6. A device according to any preceding claim wherein the transfer means is a wireless transmission means to a remote data retention means.

7. A device according to any preceding claim, wherein the device comprises a plurality of sensors.

8. A device according to any preceding claim, wherein the or each sensor obtains data readings at a rate of from 10-200 Hz.

9. A device according to claim 7, wherein data readings are obtained at a rate of around 100Hz.

## Patentansprüche

1. Vorrichtung zum Bestimmen des Ausmaßes der Dyskinesie bei einem Individuum, wobei die Vorrichtung umfasst
einen Sensor, der abnehmbar an der Extremität, dem Rumpf oder dem Kopf des Individuums befestigt werden kann, wobei der Sensor einen ersten Detektor zum Erfassen einer 3D-Bewegung beinhaltet;
Datenspeichermittel zum Speichern von durch den Detektor erzeugten Daten; Übertragungsmittel zum Übertragen der Daten an das Datenspeichermittel; Verarbeitungsmittel, um die Daten in den Datenspeichermitteln zu verarbeiten und die verarbeiteten Daten zu klassifizieren;
Ausgabemittel zum Anzeigen des diagnostizierten Zustands für einen Benutzer;
**dadurch gekennzeichnet, dass** das Verarbeitungsmittel angepasst ist, um einen evolutionären Algorithmus bei der Klassifizierung der Daten und dem Vergleich mit einem bekannten Dyskinesiezustand anzuwenden, und dadurch dass der Sensor ferner einen zweiten Bewegungsmelder zum Erfassen von Neigung, Rollen und Gieren des Sensors beinhaltet, wobei der zweite Bewegungsmelder weitere Daten an die Datenspeichermittel liefert, die auch von den Verarbeitungsmitteln verwendet zu werden, um das Ausmaß der Dyskinesie zu bestimmen.

2. Vorrichtung nach Anspruch 1, wobei der erste Bewegungsmelder einen oder mehrere Beschleunigungssensoren umfasst, um die 3D-Bewegungsdaten bereitzustellen.

3. Vorrichtung nach einem der vorhergehenden Ansprüche, wobei die Vorrichtung einen weiteren Bewegungsmelder zum Messen von Positionsdaten beinhaltet.

4. Vorrichtung nach 3, worin die Positionsdaten unter Bezugnahme auf kartesische Koordinaten bestimmt werden.

5. Vorrichtung nach Anspruch 3 oder 4, worin die Verarbeitungseinrichtung Positionsdaten in Geschwindigkeits- und/oder Beschleunigungsdaten umwandelt.

6. Vorrichtung nach einem der vorhergehenden Ansprüche, worin das Übertragungsmittel ein drahtloses Übertragungsmittel zu einem entfernten Datenspeichermittel ist.

7. Vorrichtung nach einem der vorhergehenden Ansprüche, worin die Vorrichtung eine Vielzahl von Sensoren umfasst.

8. Vorrichtung nach einem der vorhergehenden Ansprüche, worin der oder jeder Sensor Datenablesungen mit einer Rate von 10-200 Hz erhält.

9. Vorrichtung nach Anspruch 7, worin Datenablesungen mit einer Rate von etwa 100Hz erhalten werden.

## Revendications

1. Dispositif pour déterminer l'étendue de la dyskinésie chez un sujet, le dispositif comprenant
un capteur pouvant être fixé de manière amovible sur le membre, le torse ou la tête du sujet, ledit capteur comprenant un premier détecteur pour détecter un mouvement 3D ; des moyens de rétention de données pour retenir les données générées par le détecteur ; des moyens de transfert pour transférer lesdites données aux moyens de rétention des données ;
des moyens de traitement, pour traiter les données dans les moyens de rétention des données et classer lesdites données traitées ;
des moyens de sortie pour afficher l'état diagnostiqué à un utilisateur ;
**caractérisé en ce que** les moyens de traitement sont adaptés pour employer un algorithme évolutif dans la classification desdites données et la comparaison avec un état de dyskinésie connu, et **en ce que** le capteur comprend en outre un second détecteur de mouvement pour détecter le tangage, le roulis et le lacet du capteur, ledit second détecteur de mouvement fournissant des données supplémentaires aux moyens de rétention de données, qui doivent également être utilisés par les moyens de traitement pour déterminer l'étendue de la dyskinésie.

2. Dispositif selon la revendication 1, dans lequel le premier détecteur de mouvement comprend un ou plusieurs accéléromètres pour fournir lesdites données de mouvement 3D.

3. Dispositif selon l'une quelconque des revendications précédentes, dans lequel le dispositif comprend un autre détecteur de mouvement pour mesurer des données de position.

4. Dispositif selon 3, dans lequel les données de position sont déterminées par référence à des coordonnées cartésiennes.

5. Dispositif selon la revendication 3 ou 4, dans lequel lesdits moyens de traitement convertissent des données de position en données de vitesse et/ou d'accélération.

6. Dispositif selon toute revendication précédente, dans lequel les moyens de transfert sont des moyens de transmission sans fil vers des moyens de rétention de données à distance.

7. Dispositif selon l'une quelconque des revendications précédentes, dans lequel le dispositif comprend une pluralité de capteurs.

8. Dispositif selon l'une quelconque des revendications précédentes, dans lequel le ou chaque capteur obtient des lectures de données à une fréquence de 10-200 Hz.

9. Dispositif selon la revendication 7, dans lequel des lectures de données sont obtenues à une fréquence d'environ 100 Hz.
